(19) Europäisches Patentamt
European Patent Office
Office européen des brevets

(11) **EP 2 110 414 A1**

(12) **EUROPÄISCHE PATENTANMELDUNG**

(43) Veröffentlichungstag:
**21.10.2009 Patentblatt 2009/43**

(51) Int Cl.:
*C09C 1/30* (2006.01)          *C08K 9/06* (2006.01)

(21) Anmeldenummer: **08007625.0**

(22) Anmeldetag: **18.04.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Nanoresins AG**
**21502 Geesthacht (DE)**

(72) Erfinder: **Kühner, Uwe Dietrich, Dr.**
**20457 Hamburg (DE)**

(74) Vertreter: **Von Renesse, Dorothea et al**
**König Szynka Tilmann von Renesse**
**Patentanwälte Partnerschaft**
**Lohengrinstrasse 11**
**40549 Düsseldorf (DE)**

(54) **Oberflächenmodifizierte Siliziumdioxid-Partikel**

(57) Ein oberflächenmodifiziertes Siliziumdioxid-Partikel wird beschrieben, dessen Oberfläche durch die folgende Belegung gekennzeichnet ist:

(a) 0,1 bis 16 Gruppen/nm$^2$ der Art (Modifizierung vom Typ A)

$$(\text{Oberfläche-SiO})_x\text{-Si}(R^1)_y(OR^2)_{4-x-y}$$

mit x = 1 bis 3, y = 1 bis 3 und x + y = 2 oder 3; und
(b) 0,1 bis 16 Gruppen/nm$^2$ der Art (Modifizierung vom Typ B)

$$(\text{Oberfläche-SiO})_2\text{SiR}^3{}_{4-z}$$

mit z = 1 oder 2

aufweist, wobei die Reste $R^1$, $R^2$ und $R^3$ beliebige organische Reste darstellen können und mehrere Reste $R^1$, $R^2$ oder $R^3$ gleich oder verschieden sein können, und A ungleich B ist.

**Beschreibung**

Die vorliegende Erfindung betrifft modifizierte Siliziumdioxid-Partikel, sowie ein Verfahren zur Herstellung modifizierter Siliziumdioxid-Partikel und die durch dieses Verfahren erhältlichen Produkte, bestimmte Verwendungszwecke der Siliziumdioxid-Partikel und Nanocomposite, welche die Siliziumdioxid-Partikel enthalten. Ebenso werden Kieselsole enthaltend die modifizierten Siliziumdioxid-Partikel vorgeschlagen.

Kieselsol ist eine Suspension von amorphem Siliziumdioxid ($SiO_2$), in welcher das Siliziumdioxid in Form von im Wesentlichen (d.h. zu mindestens 50%, vorzugsweise zu mindestens 70, 80 oder 90%) untereinander unvernetzten, kugelförmigen Einzelpartikeln vorliegt. Das Dispersionsmittel kann verschieden sein, so. z.B. ein Lösungsmittel oder ein Monomer.

Kieselsole werden vielseitig eingesetzt. Beispielsweise sind sie für den Einsatz als Bindemittel für Feinguss, für Fasern im Feuerfestbereich und bei der Herstellung von Katalysatoren, als Beschichtungsagenzien für Folien (Antiblocking), im Textilsektor für Schiebefestausrüstungen, im Bausektor als Additive für Spritzbeton oder als Binder für Brand- und Wärmeschutzanwendungen, als Poliermittel für die Elektronik oder auch im Papiersektor, beispielsweise bei der Papierretention oder als Additiv in der Beschichtung von Spezialpapieren, geeignet.

Herkömmliche Kieselsole sind, je nach Teilchengröße der Siliziumdioxid-Partikel, milchig trüb über opaleszierend bis farblos klar ausgebildet. Die Partikel haben im Allgemeinen Durchmesser von 5 nm bis 150 nm und sind in der Regel kugelförmig, räumlich begrenzt und vorzugsweise elektrisch negativ geladen. Im Innern der einzelnen Partikel liegt üblicherweise ein Gerüst von Siloxanbindungen vor, welches sich aus der Verknüpfung von $[SiO_4]$-Tetraedem bzw. von Polykieselsäuren ergibt.

Aufgrund ihrer geringen Größe haben die Partikel eine große spezifische Oberfläche, was wiederum zu einer hohen Oberflächenenergie führt. Eine unerwünschte Folge dieser hohen Oberflächenenergie ist, dass die Partikel dazu neigen, Agglomerate oder sogar Aggregate zu bilden. Damit ist die Bildung stabiler, Siliziumdioxid-Partikel enthaltender Dispersionen nicht ohne weiteres möglich.

Um die Agglomeration oder die Aggregation der Partikel möglichst zu verhindern, ist aus dem Stand der Technik bekannt, sie an ihrer Oberfläche zu modifizieren.

So ist aus "The Chemistry of Silica" von Ralph K. Iler (1979, John Wiley & Sons, Inc.; New York, Chichester, Brisbane, Toronto) bekannt, Silikatoberflächen mit Chlorsilanen zu modifizieren.

EP 0 982 268 A beschreibt die Umsetzung wässriger Kieselsole in Anwesenheit wassermischbarer Lösemittel mit Halogensilanen und Mischungen aus Siloxanen und Halogensilanen bzw. Siloxanen.

US 6,736,891 beschreibt die Umsetzung einer wässrigen Suspension von Fällungskieselsäuren bei einem niedrigen pH-Wert mit Hexamethyldisiloxan in Gegenwart von Isopropanol.

Die aus dem Stand der Technik bekannten modifizierten Siliziumdioxid-Partikel bzw. deren Herstellungsverfahren weisen nur eine unzureichende Flexibilität auf. So ist die Anpassung der Partikel für die Weiterverarbeitung zu Kompositen nur eingeschränkt möglich.

Aus der US 2,801,185 ist ein organisches oberflächenmodifiziertes Siliziumdioxid-Partikel bekannt sowie ein Verfahren zu dessen Herstellung, wobei ein Wasser enthaltendes Kieselsol mit einem organischen Lösemittel versetzt und das Wasser azeotrop entfernt wird, so dass der Wasseranteil auf unter 1 % herabgesetzt wird. Anschließend wird ein Modifizierungsmittel (Beschichtungsmaterial) zugegeben und die Modifikation der Oberfläche vorgenommen. Als Beschichtungsmittel werden unter anderem gesättigte primäre und sekundäre Alkohole erwähnt. Diese Partikel haben aber den Nachteil, dass sie nicht die gewünschte Stabilität aufweisen und/oder z.T. nicht die gewünschte Flexibilität in der Weiterverarbeitung erlauben.

Aus der US 2,786,042 ist bekannt, organische Kieselsole mit kohlenwasserstoffhaltigen Silanolen an der Oberfläche zu modifizieren.

Nachteilig an diesen bekannten Verfahren ist demnach, dass daraus modifizierte Partikel resultieren, welche nur eine bedingte Redispergierbarkeit aufweisen. Darüber hinaus weisen sie eine nur eingeschränkte Verträglichkeit mit organischen Lösemitteln, wie Toluol und Hexan, oder organischen Harzen und Polymeren, auf. Außerdem lassen sich die Partikel nur in engen Grenzen verändern, so dass eine flexible Anpassung ist nicht möglich.

Die Aufgabe der vorliegenden Erfindung besteht somit in der Bereitstellung oberflächenmodifizierter Kieselsolteilchen (Siliziumdioxid-Partikeln), welche eine verbesserte Redispergierbarkeit oder bessere Verträglichkeit/Komptabilität in bestimmten organischen Lösemitteln zeigen, insbesondere in Toluol.

Gelöst wird diese Aufgabe durch Siliziumdioxid-Partikel, dessen Oberfläche durch einen Belegungsgrad wie folgt modifiziert ist

(a) 0,1 bis 16 Gruppen/nm$^2$ der Art (Modifizierung vom Typ A)

$$(\text{Oberfläche-SiO})_x\text{-Si}(R^1)_y(OR^2)_{4-x-y}$$

mit x = 1 bis 3, y = 1 bis 3 und x + y = 2 oder 3; und

(b) 0,1 bis 16 Gruppen/nm$^2$ der Art (Modifizierung vom Typ B)

$$(\text{Oberfläche-SiO})_z SiR^3{}_{4-z}$$

mit z = 1 oder 2

aufweist, wobei die Reste $R^1$, $R^2$ und $R^3$ beliebige organische Reste darstellen können und mehrere Reste $R^1$, $R^2$ oder $R^3$ gleich oder verschieden sein können.

Erfindungsgemäß wurde herausgefunden, dass die derart an der Oberfläche modifizierten Partikel eine hervorragende Redispergierbarkeit in organischen Lösemitteln und eine hervorragende Verträglichkeit mit Lösemitteln, wie Toluol, aufweisen. Dieses liegt vorrangig darin begründet, dass ihre Oberfläche durch die zuvor beschriebene Modifizierung mit organischen Resten versehen wird. Wesentlich dabei ist, dass die Partikel tatsächlich die beiden erfindungsgemäß verschiedenen Modifizierungen nach A und B aufweisen. Die Erfindung erfasst demnach keine Varianten, in denen die Modifizierung vom Typ A gleich der Modifizierung vom Typ B ist. Die Modifizierung vom Typ A ist daher ungleich der Modifizierung vom Typ B (A≠B).

Die erfindungsgemäßen Siliziumdioxid-Partikel weisen eine Modifizierung der Art (Oberfläche-SiO)$_x$-Si(R)$_y$(OR)$_{4-x-y}$(Modifizierungsart vom Typ A) von vorzugsweise 0,1 bis 16, vorzugsweise 0,1 bis 10 Gruppen/nm$^2$, insbesondere 0,15 bis 6 Gruppen/nm$^2$, besonders bevorzugt 0,2 bis 4 Gruppen/nm$^2$, auf.

Ferner weisen sie eine Modifizierung der Art (Oberfläche-SiO)$_z$-SiR$^3{}_{4-z}$ (Modifizierungsart vom Typ B) von 0,1 bis 16, vorzugsweise 0,2 bis 10 Gruppen/nm$^2$, insbesondere 0,3 bis 6 Gruppen/nm$^2$, besonders bevorzugt 0,4 bis 4 Gruppen/nm$^2$, auf.

Die vorstehend genannten Vorzugsbereiche für Modifizierungsarten vom Typ A und B können beliebig miteinander kombiniert werden. Die konkrete Kombination hängt von den Notwendigkeiten der weiteren Einsatzbereiche und der Weiterverarbeitung der Poartikel ab. Bevorzugt ist eine Kombination von insbesondere 0,9 - 3,6 Gruppen/nm$^2$ der Modifizierung vom Typ A und 0,5 - 3 Gruppen/nm$^2$ der Modifizierung vom Typ B.

Dem Fachmann sind Methoden zur Bestimmung von funktionalen Gruppen auf der Oberfläche der Partikel, und somit auch der Belegung der erfindungsgemäßen Partikel, bekannt. So lassen sich Gruppen aus $R_2Si$ und $R_3Si$ mit Hilfe von Basen (z.B. Kaliumhydroxyd) abspalten, die dann Disiloxane ($R_3SiOSiR_3$) oder Cyclen ($R_2SiO$)n bilden. Diese lassen sich im GC analysieren. Die Methode ist beispielsweise in EP 0982268 B1 erwähnt (Vergleichsbeispiel und Beispiel 1). Auch sind die Gruppen grundsätzlich über NMR und IR bestimmbar. Vinylgruppen lassen sich außerdem über die sog. Iodzahl titrimetrisch erfassen, nämlich durch die Umsetzung der Vinylgruppen mit sog. Wijs-Lösung und anschließender Titration des überschüssigen Halogens mit Natriumthiosulfat. Die Bestimmung von (Meth)acryloylgruppen kann mit Differential Scanning Calorimetry (DSC) bei der Umsetzung mit standardisierten Peroxidlösungen anhand der freiwerdenden Reaktionswärme erfolgen.

## Modifizierung vom Typ A

Die Modifizierung der Partikeloberfläche vom Typ A erfolgt durch Umsetzung von Kieselsolen mit Alkoxysilanen der allgemeinen Formel (I)

$$\text{Formel (I)} \qquad R^1{}_x Si(OR^2)_{4-x},$$

in welchen der Rest $R^1$ einem gegebenenfalls substituierten oder funktionalisierten $C_1$-$C_{18}$-Alkylrest entspricht und der Rest $R^2$ ausgewählt sein kann aus der Gruppe, bestehend aus einem gegebenenfalls substituierten oder funktionalisierten $C_1$-$C_{18}$-Alkylrest, einem Carboxyrest, einem gegebenenfalls substituierten $C_2$-$C_{18}$-Alkenylrest und einem Oximrest.

Im Rahmen der vorliegenden Erfindung werden die Verbindungen der allgemeinen Formel (I) vorzugsweise ausgewählt aus der Gruppe, bestehend aus Methyltrimethoxysilan, Trimethylmethoxysilan, Methylhydrogendimethoxysilan, Dimethyldimethoxysilan, Ethyltrimethoxysilan, Ethyltriacetoxysilan, Propyltrimethoxysilan, Diisopropyldimethoxysilan, Diisobutyldimethoxysilan, Chlorpropyltrimethoxysilan, Chlorpropylmethyldimethoxysilan, Chlorisobutylmethyldimethoxysilan, Trifluorpropyltrimethoxysilan, Trifluorpropylmethyldimethoxysilan, iso-Butyltrimethoxysilan, n-Butyltrimethoxysilan, n-Butylmethyldimethoxysilan, Phenyltrimethoxysilan, Phenyltrimethoxysilan, Phenylmethyldimethoxysilan, Triphenylsilanol, n-Hexyltrimethoxysilan, n-Octyltrimethoxysilan, iso-Octyltrimethoxysilan, Decyltrimethoxysilan, Hexadecyltrimethoxysilan, Cyclohexylmethyldimethoxysilan, Cyclohexylethyldimethoxysilan, Dicyclopentyldimethoxysilan, tert.-Butylethyldimethoxysilan, tert.-Butylpropyldimethoxysilan, Dicyclohexyldimethoxysilan, Mercaptopropyltrimethoxysilan, Mercaptopropylmethyldimethoxysilan, Bis(triethoxysilylpropyl)disulfid, Bis(triethoxysilylpropyl)tetrasulfid, Aminopropyltrimethoxysilan, m-Aminophenyltrimethoxysilan, Aminopropylmethyldiethoxysilan, Phenylaminopropyltrimethoxysilan, Aminoethylaminopropyltrimethoxysilan, Aminoethylaminopropylmethyldimethoxysilan, Glycidoxypropyltrimethoxysilan, Glycidoxypropylmethyldimethoxysilan, Epoxycyclohexylethyltrimethoxysilan, $\gamma$-Methacryloxypropyltriacetoxysilan, Vinyltriacetoxysilan, Vinyltrimethoxysilan, Methylvinyldimethoxysilan, Vinyldimethylmethoxysilan, Divinyldimethoxysilan, Vinyltris(2-methoxyethoxy)silan, Hexenyltrimethoxysilan, $\gamma$-Methacroyloxypropyltrimethoxysilan, Acryloxypropyltrimethoxysilan, Vinylbenzylethylendiaminpropyltrimethoxysilan, Vinylbenzylethylendiaminpropyltrimethoxysilan-Hydrochlorid, Allylethylendiaminpropyltrimethoxysilan, Allyltrimethoxysilan, Allylmethyldimethoxysilan, Allyldimethylmethoxysilan und Hexenyltrimethoxysilan.

Im Rahmen der vorliegenden Erfindung sind insbesondere Silane der allgemeinen Formel (I-1)

$$\text{Formel (I-1)} \qquad R^1Si(OR^2)_3 \ (x=3)$$

bevorzugt, wobei die Reste $R^1$ und $R^2$ die vorgenannte Bedeutung aufweisen.

### Modifizierung vom Typ B

Die Modifizierung der Partikeloberfläche vom Typ B wird über eine Umsetzung mit einem Halogensilan und/oder einem Siloxan durchgeführt.

Die Halogensilane weisen dabei vorzugsweise die allgemeine Formel (II)

$$\text{Formel (II)} \qquad R^3{}_aH_bSiX_{4-a-b}$$

auf, worin

jedes $R^3$, unabhängig voneinander, ausgewählt ist aus der Gruppe, bestehend aus Kohlenwasserstoffresten mit 1 bis 18 Kohlenstoffatomen oder organofunktionellen Kohlenwasserstoffresten mit 1 bis 18 Kohlenstoffatomen;
X, unabhängig voneinander, ausgewählt ist aus der Gruppe, bestehend aus Fluor, Chlor, Brom und Iod;
a gleich 0, 1, 2 oder 3 ist;
b gleich 0 oder 1 ist; und
a + b = 1, 2 oder 3 ist.

Bevorzugt sind im Rahmen der vorliegenden Erfindung insbesondere Chlorsilane der allgemeinen Formel (II-1)

$$\text{Formel (II-1)} \qquad R^3{}_aH_bSiCl_{4-a-b},$$

wobei der Rest $R^3$ und die Indizes a und b die vorgenannte Bedeutung aufweisen.

Besonders bevorzugt sind im Rahmen der vorliegenden Erfindung Halogensilane der allgemeinen Formel (II-2)

$$\text{Formel (II-2)} \qquad R^3{}_aH_{3-a}SiCl,$$

wobei der Rest $R^3$ und der Index a die vorgenannte Bedeutung aufweisen.

Im Rahmen der vorliegenden Erfindung werden die Verbindungen der allgemeinen Formel (II) vorzugsweise ausgewählt aus der Gruppe, bestehend aus Chlortrimethylsilan, Bromtrimethylsilan, Iodtrimethylsilan, Dichlordimethylsilan, Dichlormethylsilan, Methyltrichlorsilan, Chlordimethylsilan, Trichlorsilan, Ethyltrichlorsilan, Propyltrichlorsilan, Phenyltrichlorsilan, Dichlordiphenylsilan, n-Hexyltrichlorsilan, n-Octyltrichlorsilan, Chlordimethyloctylsilan, Chlordimethyloctadecylsilan, Vinylttichlorsilan, Dichlormethylvinylsilan, Chlordimethylvinylsilan, Dichlordivinylsilan, $\gamma$-Methacryloxypropyldimethylchlorsilan, Allyltrichlorsilan, Allyldichlormethylsilan und Allylchlordimethylsilan.

Die Siloxane weisen die bevorzugte allgemeine Struktur (III)

$$\text{Formel (III)} \qquad R^3{}_nSiO_{(4-n)/2}$$

auf, worin

jedes $R^3$, unabhängig voneinander, ausgewählt ist aus der Gruppe, bestehend aus Kohlenwasserstoffresten mit 1 bis 18 Kohlenstoffatomen, organofunktionellen Kohlenwasserstoffresten mit 1 bis 18 Kohlenstoffatomen, einem Wasserstoffatom und einer OH-Gruppe; und n eine Zahl zwischen 2 und einschließlich 3 ist.

Bevorzugt sind im Rahmen der vorliegenden Erfindung Siloxane der allgemeinen Formel (III-1)

$$\text{Formel (III-1)} \qquad R^3{}_3SiOSiR^3{}_3,$$

wobei der Rest $R^3$ die vorgenannte Bedeutung aufweist und mehrere $R^3$ jeweils eine unterschiedliche Bedeutung aufweisen können.

Weiter bevorzugt sind im Rahmen der vorliegenden Erfindung cyclische Siloxane der allgemeinen Formel (III-2)

$$\text{Formel (III-2)} \qquad (R^3_2SiO)_n,$$

wobei n eine ganze Zahl ist und mehrere $R^3$ jeweils eine unterschiedliche Bedeutung aufweisen können.

Weiter bevorzugt sind im Rahmen der vorliegenden Erfindung Polysiloxane der allgemeinen Formel (III-3)

$$\text{Formel (III-3)} \qquad R^3_3SiO(R^3_2SiO)_nSiR^3_3,$$

wobei n eine ganze Zahl ist und mehrere $R^3$ jeweils eine unterschiedliche Bedeutung aufweisen können.

In einer bevorzugten Ausführungsform wird ein Polysiloxan der Verbindung III-1 eingesetzt.

Im Rahmen der vorliegenden Erfindung werden die Verbindungen der allgemeinen Formel (III) vorzugsweise ausgewählt aus der Gruppe, bestehend aus Hexamethyldisiloxan, Octamethyltrisiloxan, Hexamethylcyclotrisiloxan, Octamethylcyclotetrasiloxan, Decamethylcyclopentasiloxan, Tetramethyldisiloxan, Trimethylcyclotrisiloxan, Tetramethylcyclotetrasiloxan, Pentamethylcyclopentasiloxan, Divinyltetramethylsiloxan, Trimethyltrivinylcyclosiloxan und Tetramethyltetravinylcyclotetrasiloxan.

### Herstellung der erfindungsgemäßen Partikel bzw. Kieselsole

Die erfindungsgemäßen Partikel können beispielsweise durch die Umsetzung von Kieselsol mit mindestens einem ersten Modifizierungsmittel, umfassend mindestens ein Alkoxysilan, und mindestens einem zweiten Modifizierungsmittel, ausgewählt aus der Gruppe bestehend aus einem Halogensilan, einem Siloxan und Mischungen davon, hergestellt werden.

Als Edukt für die erfindungsgemäßen Siliziumdioxid-Partikel kommen Dispersionen von kolloidalem Siliziumdioxid oder Lösungsmitteln in Frage. Dabei kann ein kolloidales Siliziumdioxid verwendet werden, welches beispielsweise nach der Stöber-Synthese oder aus Wasserglas hergestellt wurde. Unter kolloidalem Siliziumdioxid werden im Sinne der vorliegenden Erfindung Partikel mit einem durchschnittlichen Durchmesser von $\geq 1$ bis 1000 nm, vorzugsweise von 5 bis 150 nm verstanden. Sie können dispergiert in einer Flüssigkeit vorliegen (Kieselsol). Die Partikel bestehen im wesentlichen, nämlich vorzugsweise zu mindestens 90%, besonders bevorzugt zu mindestens 95 oder 99% aus Siliziumdioxid.

Bei der Stöber-Synthese werden Alkoxysilane, wie Tetramethoxysilan, in Anwesenheit von Säuren oder Basen als Katalysator hydrolysiert und dadurch gezielt Teilchen aufgebaut. Der Vorteil dieses Verfahrens besteht darin, dass sich sehr enge Teilchengrößenverteilungen und eine beliebige Teilchengröße zwischen 5 und 500 nm erreichen lassen.

Bei der Herstellung von kolloidalem Kieselsol ausgehend von Wasserglas wird eine wässrige Lösung von Natriumsilikat mittels eines Ionenaustauschers entionisiert, wodurch Kieselsäure $(Si(OH)_4)$ gebildet wird. Die entstehende Kieselsäure ist im Allgemeinen instabil und polymerisiert unmittelbar zu kleinen Saat-Partikeln, aus denen dann die eigentlichen Partikel aufgebaut werden. Durch ein geeignetes Einstellen der Prozessbedingungen können enge Partikelgrößenverteilungen im Bereich von beispielsweise etwa 5 bis 150 nm hergestellt werden. Die wässrigen Kieselsole werden im Allgemeinen mit Basen stabilisiert, wodurch die sauren Kieselsäurepartikel negativ geladen werden und sich abstoßen. Wenn im Rahmen der vorliegenden Erfindung ein Kieselsol als Ausgangsmaterial verwendet wird, welches beispielsweise herstellungsbedingt eine Base enthält, wird diese Base jedoch vorzugsweise zunächst entfernt.

Auch kommerziell erhältliche Siliziumdioxid-Partikel, beispielsweise Bindzil 40/130 und Bindzil 40/220 (erhältlich von Eka Chemicals); Levasil 200/40% (erhältlich von H.C. Starck); Nalco 2327, Nalco 1144 und Nalco 2329 (erhältlich von Nalco Company); NexSil 12 und NexSil 20 (erhältlich von Nyacol); Snowtex ST-40 und Snowtex ST-50 (erhältlich von Nissan Chemical American Corporation) können verwendet werden.

Das erfindungsgemäße Verfahren geht somit bevorzugt von einem nanoskaligen, kolloidalen Kieselsäuresol aus. Der pH-Wert dieses Sols wird bevorzugt auf 5 oder weniger, weiter bevorzugt auf 4 oder weniger eingestellt. Bei einem basischen Sol kann dies durch Zugabe von Säure oder durch Verwendung eines sauren Kationenaustauschers geschehen.

Die Umsetzung mit dem ersten und dem zweiten Modifizierungsmittel kann sowohl aufeinanderfolgend als auch gleichzeitig mit einer Mischung aus dem ersten und dem zweiten Modifizierungsmittel erfolgen.

Im Rahmen des erfindungsgemäßen Verfahrens wird aus dem Kieselsol zu einem beliebigen Zeitpunkt des Verfahrensablaufs vorzugsweise Wasser entfernt, da größere Mengen an Wasser im Reaktionsgemisch dazu führen, dass das Reaktionssystem insgesamt polarer ist. Wenn, wie im vorliegenden erfindungsgemäßen Verfahren vorzugsweise vorgesehen, Wasser aus dem Reaktionssystem entfernt wird, führt dieses zu der Möglichkeit, mit einer einem höheren Gehalt von beispielsweise bis zu 15 Gew.-%, besonders bevorzugt bis 20 Gew.-%, insbesondere bis 25 Gew.-%, des Kieselsols zu arbeiten. Damit lässt sich durch das erfindungsgemäße Verfahren eine deutlich höhere Raum-Zeit-Ausbeute realisieren.

Darüber hinaus ist es bekannt, dass Wasser die Agglomeration von Silika-Teilchen fördert. Deshalb ist es bevorzugt, Wasser aus dem Reaktionssystem des Kieselsols zu entfernen.

In einer Ausführungsform der vorliegenden Erfindung umfasst das erfindungsgemäße Verfahren damit sowohl die Verfahrensschritte

(1) der Umsetzung von kolloidalem Kieselsol mit mindestens einem ersten Modifizierungsmittel, umfassend mindestens ein Alkoxysilan,
(2) der Umsetzung von kolloidalem Kieselsol mit mindestens einem zweiten Modifizierungsmittel, ausgewählt aus einem Halogensilan, einem Siloxan und Mischungen davon, sowie
(3) die Entfernung von Wasser aus dem Kieselsol, insbesondere durch azeotrope Destillation.

Die dabei vorgesehene Reihenfolge der einzelnen Verfahrensschritte (1) bis (3) ist nicht beschränkt und variabel. So ist es im Rahmen der vorliegenden Erfindung grundsätzlich möglich, bei einer zweistufigen Modifizierung der Oberfläche von einem Kieselsol das Wasser vor der ersten Oberflächenmodifizierung oder zwischen der ersten und der zweiten Oberflächenmodifizierung aus dem Reaktionssystem zu entfernen.

In einer bevorzugten Ausführungsform des erfindungsgemäßen Verfahrens erfolgt jedoch die Umsetzung des kolloidalen Kieselsols zunächst mit mindestens einem ersten Modifizierungsmittel, umfassend mindestens ein Alkoxysilan (Verfahrensschritt (1)), vor der Entfernung von Wasser aus dem Kieselsol (3), an welche sich die Umsetzung mit mindestens einem zweiten Modifizierungsmittel, ausgewählt aus einem Halogensilan, einem Siloxan und Mischungen davon (Verfahrensschritt (2)), anschießt.

Im folgenden werden die einzelnen Verfahrensschritte (1) bis (3) näher beschrieben, wobei - wie bereits ausgeführt - die Bezeichnung die Nummerierung der jeweiligen Verfahrensschritte keine Beschränkung der Reihenfolge der Verfahrensschritte bedeutet.

**Verfahrensschritt (1)**

Die Modifizierung der Partikeloberfläche gemäß dem Verfahrensschritt (1) kann erfolgen durch die Umsetzung von Kieselsolen mit Alkoxysilanen der allgemeinen Formel (I)

$$\text{Formel (I)} \qquad R^1_x Si(OR^2)_{4-x},$$

in welchen der Rest $R^1$ einem gegebenenfalls substituierten $C_1$-$C_{18}$-Alkylrest entspricht und der Rest $R^2$ ausgewählt sein kann aus der Gruppe, bestehend aus einem gegebenenfalls substituierten $C_1$-$C_{18}$-Alkylrest, einem Carboxyrest, einem gegebenenfalls substituierten $C_2$-$C_{18}$-Alkenylrest und einem Oximrest.

Beachtlich ist, dass durch eine Hydrolyse der entstehenden SiOR Gruppen SiOH Gruppen entstehen können, an denen wiederum Alkyoxysilane anlagern können. Damit können ganz oder teilweise Schichten entstehender, die Si(R)-x-O-Si(R)x-Verknüpfungen aufweisen.

Im Rahmen der vorliegenden Erfindung werden die Verbindungen der allgemeinen Formel (I) vorzugsweise ausgewählt aus der Gruppe, bestehend aus Methyltrimethoxysilan, Trimethylmethoxysilan, Methylhydrogendimethoxysilan, Dimethyldimethoxysilan, Ethyltrimethoxysilan, Ethyltriacetoxysilan, Propyltrimethoxysilan, Diisopropyldimethoxysilan, Diisobutyldimethoxysilan, Chlorpropyltrimethoxysilan, Chlorpropylmethyldimethoxysilan, Chlorisobutylmethyldimethoxysilan, Trifluorpropyltrimethoxysilan, Trifluorpropylmethyldimethoxysilan, iso-Butyltrimethoxysilan, n-Butyltrimethoxysilan, n-Butylmethyldimethoxysilan, Phenyltrimethoxysilan, Phenyltrimethoxysilan, Phenylmethyldimethoxysilan, Triphenylsilanol, n-Hexyltrimethoxysilan, n-Octyltrimethoxysilan, iso-Octyltrimethoxysilan, Decyltrimethoxysilan, Hexadecyltrimethoxysilan, Cyclohexylmethyldimethoxysilan, Cyclohexylethyldimethoxysilan, Dicyclopentyldimethoxysilan, tert.-Butylethyldimethoxysilan, tert.-Butylpropyldimethoxysilan, Dicyclohexyldimethoxysilan, Mercaptopropyltrimethoxysilan, Mercaptopropylmethyldimethoxysilan, Bis(triethoxysilylpropyl)disulfid, Bis(triethoxysilylpropyl)tetrasulfid, Aminopropyltrimethoxysilan, m-Aminophenyltrimethoxysilan, Aminopropylmethyldiethoxysilan, Phenylaminopropyltrimethoxysilan, Aminoethylaminopropyltrimethoxysilan, Aminoethylaminopropylmethyldimethoxysilan, Glycidoxypropyltrimethoxysilan, Glycidoxypropylmethyldimethoxysilan, Epoxycyclohexylethyltrimethoxysilan, γ-Methacryloxypropyltriacetoxysilan, Vinyltriacetoxysilan, Vinyltrimethoxysilan, Methylvinyldimethoxysilan, Vinyldimethylmethoxysilan, Divinyldimethoxysilan, Vinyltris(2-methoxyethoxy)silan, Hexenyltrimethoxysilan, γ-Methacroyloxypropyltrimethoxysilan, Acryloxypropyltrimethoxysilan, Vinylbenzylethylendiaminpropyltrimethoxysilan, Vinylbenzylethylendiaminpropyltrimethoxysilan-Hydrochlorid, Allylethylendiaminpropyltrimethoxysilan, Allyltrimethoxysilan, Allylmethyldimethoxysilan, Allyldimethylmethoxysilan und Hexenyltrimethoxysilan.

Im Rahmen der vorliegenden Erfindung sind insbesondere Silane der allgemeinen Formel (I-1)

Formel (I-1)        $R^1Si(OR^2)_3$ (x=3)

bevorzugt, wobei die Reste $R^1$ und $R^2$ die vorgenannte Bedeutung aufweisen.

Der Verfahrensschritt (1) wird vorzugsweise bei den folgenden Bedingungen durchgeführt: Für Verfahrensschritt (1) wird das Kieselsol sofern es basisch stabilisiert ist, mit einem Kationentauscher behandelt. Das saure Kieselsol wird dann zur Reaktion gebracht.

Ist das Silan entsprechend Formel (I-1) ausreichend löslich in dem Kieselsol, erfolgt die Reaktion bevorzugt bei Raumtemperatur innerhalb von zwei Stunden. Ist das Silan nicht ausreichend löslich im Kieselsol (erkennbar an Fettaugen nach 15 Minuten intensivem Mischen), muss die Mischung mit einem wassermischbaren Lösungsmittel verdünnt werden. Besonders bevorzugt ist dafür Isopropanol oder 1-Methoxy-2-propanol. Die Mischung mit dem Silan kann auch erwärmt werden.

Die bevorzugte Menge Silan lässt sich anhand der spezifischen Partikeloberfläche $A_0$ berechnen. Es werden bevorzugt 1,5 - 6*$\mu$mol(Silan)/g(SiO$_2$) * $A_0$ eingesetzt. Je größer die Partikel sind desto kleiner ist die spezifische Oberfläche und umso geringer ist die benötigte Menge Silan. Als spezifische Oberfläche kann die Oberfläche nach der BET-Methode oder nach der unten beschriebenen Methode auf Basis der Teilchengröße heran gezogen werden.

Unter diesen Bedingungen reagiert das Silan vollständig mit der Partikeloberfläche, so dass die Beladung mit Gruppen im wesentlichen der eingesetzten Stöchiometrie entspricht.

### Verfahrensschritt (2)

Die Modifizierung der Kieselsoloberfläche gemäß dem Verfahrensschritt (2) erfolgt beispielsweise durch die Umsetzung des Kieselsols mit einem Halogensilan und/oder einem Siloxan.

Die Halogensilane weisen dabei vorzugsweise die allgemeine Formel (II)

Formel (II)        $R^3_aH_bSiX_{4-a-b}$

auf, worin

jedes $R^3$, unabhängig voneinander, ausgewählt ist aus der Gruppe, bestehend aus Kohlenwasserstoffresten mit 1 bis 18 Kohlenstoffatomen oder organofunktionellen Kohlenwasserstoffresten mit 1 bis 18 Kohlenstoffatomen;

X, unabhängig voneinander, ausgewählt ist aus der Gruppe, bestehend aus Fluor, Chlor, Brom und Iod;

a gleich 0, 1, 2 oder 3 ist;

b gleich 0 oder 1 ist; und

a + b = 1, 2 oder 3 ist.

Die für das erfindungsgemäße Verfahren einsetzbaren Halogensilane weisen vorteilhafterweise die Eignung auf, Partikel mit der Modifizierung des Typ B zu erzeugen. Dazu ist a bevorzugt 1-3, besonders bevorzugt 2 oder 3. Besonders bevorzugt wird ein Halogensilan, weiter vorzugsweise ein Chlorsilan verwendet. Die Silane können funktionalisiert sein, beispielsweise mit polymerisierbaren Gruppen, insbesondere Vinylgruppen.

Bevorzugt sind im Rahmen der vorliegenden Erfindung Chlorsilane der allgemeinen Formel (II-1)

Formel (II-1)        $R^3_aH_bSiCl_{4-a-b}$,

wobei der Rest $R^3$ und die Indizes a und b die vorgenannte Bedeutung aufweisen.

Besonders bevorzugt sind im Rahmen der vorliegenden Erfindung Halogensilane der allgemeinen Formel (II-2)

Formel (II-2)        $R^3_aH_{3-a}SiCl$,

wobei der Rest $R^3$ und der Index a die vorgenannte Bedeutung aufweisen.

Im Rahmen der vorliegenden Erfindung werden die Verbindungen der allgemeinen Formel (II) vorzugsweise ausgewählt aus der Gruppe, bestehend aus Chlortrimethylsilan, Bromtrimethylsilan, Iodtrimethylsilan, Dichlordimethylsilan, Dichlormethylsilan, Methyltrichlorsilan, Chlordimethylsilan, Trichlorsilan, Ethyltrichlorsilan, Propyltrichlorsilan, Phenyltrichlorsilan, Dichlordiphenylsilan, n-Hexyltrichlorsilan, n-Octyltrichlorsilan, Chlordimethyloctylsilan, Chlordimethyloctadecylsilan, Vinyltrichlorsilan, Dichlormethylvinylsilan, Chlordimethylvinylsilan, Dichlordivinylsilan, γ-Methacryloxypropyldimethylchlorsilan, Allyltrichlorsilan, Allyldichlormethylsilan und Allylchlordimethylsilan.

Die Siloxane weisen die bevorzugte allgemeine Struktur (III)

Formel (III)        $R^3_nSiO_{(4-n)/2}$

auf, worin

jedes $R^3$, unabhängig voneinander, ausgewählt ist aus der Gruppe, bestehend aus Kohlenwasserstoffresten mit 1 bis 18 Kohlenstoffatomen, organofunktionellen Kohlenwasserstoffresten mit 1 bis 18 Kohlenstoffatomen, einem Wasserstoffatom und einer OH-Gruppe; und n eine Zahl zwischen 2 und einschließlich 3 ist.

Bevorzugt sind im Rahmen der vorliegenden Erfindung Disiloxane der allgemeinen Formel (III-1)

Formel (III-1) $\quad\quad R^3_3SiOSiR^3_3,$

wobei $R^3$ die zuvor angegebene Bedeutung aufweist und mehrere $R^3$ jeweils eine unterschiedliche Bedeutung aufweisen können.

Weiter bevorzugt sind im Rahmen der vorliegenden Erfindung cyclische Siloxane der allgemeinen Formel (III-2)

Formel (III-2) $\quad\quad (R^3_2SiO)_n,$

wobei n eine ganze Zahl ist und mehrere $R^3$ jeweils eine unterschiedliche Bedeutung aufweisen können.

Weiter bevorzugt sind im Rahmen der vorliegenden Erfindung Polysiloxane der allgemeinen Formel (III-3)

Formel (III-3) $\quad\quad R^3_3SiO(R^3_2SiO)_nSiR^3_3,$

wobei n eine ganze Zahl ist und mehrere $R^3$ jeweils eine unterschiedliche Bedeutung aufweisen können.

Im Rahmen der vorliegenden Erfindung werden die Verbindungen der allgemeinen Formel (III) vorzugsweise ausgewählt aus der Gruppe, bestehend aus Hexamethyldisiloxan, Octamethyltrisiloxan, Hexamethylcyclotrisiloxan, Octanmethylcyclotetrasiloxan, Decamethylcyclopentasiloxan, Tetramethyldisiloxan, Trimethylcyclotrisiloxan, Tetramethylcyclotetrasiloxan, Pentamethylcyclopentasiloxan, Divinyltetramethylsiloxan, Trimethyltrivinylcyclosiloxan und Tetramethyltetravinylcyclotetrasiloxan.

Wenn die Modifizierung des Kieselsols im Verfahrensschritt (2) nur mit einem Siloxan und insbesondere ohne die gleichzeitige Verwendung von mindestens einem Halogensilan durchgeführt wird, ist es weiter bevorzugt, dass während der Umsetzung zusätzlich eine Säure verwendet wird. Die Umsetzung von Kieselsolen mit einem Halogensilan bzw. einem Siloxan basiert darauf, dass die bei der Hydrolyse bzw. Alkoholyse gebildeten Siloxane bzw. Alkoxysilane mit Hilfe von Säuren wieder gespalten und in reaktivere Produkte überführt werden können. Wenn man Halogensilane, wie Chlortrimethylsilan, in wässrigen Medien einsetzt, entsteht neben der Reaktion mit SiOH-Gruppen auf der Partikeloberfläche auch noch Hexamethyldisiloxan und Salzsäure. Die Salzsäure wiederum kann im Gleichgewicht Si-O-Si-Bindungen spalten und damit das Hexamethyldisiloxan wieder in das Chlortrimethylsilan zurück führen. Deshalb kann man ein Gemisch aus Halogensilanen und Siloxanen, Halogensilan allein oder auch ein Gemisch aus Siloxanen mit Säure, wie Salzsäure, einsetzen.

Als Säure wird in diesem Zusammenhang eine beliebige Brønstedt-Säure (wie beispielsweise in J. Huheey, Anorganische Chemie, Walter de Gruyter, Berlin, New York, 1988 S. 309f. beschrieben) verwendet. Da jedoch viele Säuren wie Salzsäure eine stark korrosive Wirkung zum Beispiel gegenüber den Materialien der Vorrichtung, in welchen das erfindungsgemäße Verfahren durchgeführt wird, aufweisen, ist ihr Einsatz auf korrosionsfeste Anlagen beschränkt. Erfindungsgemäß wird diese Problematik, die mit der Verwendung einer Bronstedt-Säure verbunden ist, vorzugsweise dadurch gelöst, dass anstelle oder zusätzlich zu der Bronstedt-Säure eine Lewis-Säure verwendet wird. Eine mögliche Lewis-Säure ist dabei Aluminiumchlorid.

Wenn im Rahmen des erfindungsgemäßen Verfahrens eine Bronstedt-Säure oder eine Lewis-Säure verwendet wird, wird diese vorzugsweise nach Verwendung aus dem oberflächenmodifizierten Kieselsol entfernt bzw. neutralisiert.

Durch die Menge an dem Modifizierungsmittel in dem zweiten Verfahrensschritt, die Temperatur der Umsetzung und Reaktionsdauer der Umsetzung lassen sich die Eigenschaften der resultierenden Kieselsole, wie die Polarität und die Redispergierbarkeit, steuern.

Der Verfahrensschritt (2) wird vorzugsweise bei den folgenden Bedingungen durchgeführt: Bevorzugt wird die Reaktion mit Chlorsilanen oder einer Mischung aus Chlorsilanen und Siloxanen durchgeführt. Bei den Siloxanen sind insbesondere die Disiloxane bevorzugt, da überschüssige Disiloxane nach der Reaktion aus dem Gemisch über eine Destillation abgetrennt werden können.

Die Reaktion wird bevorzugt im organischen Medium bei einem Wassergehalt zwischen 1 und 10 % durchgeführt. Die bevorzugte Reaktionstemperatur liegt wenig unter der Siedetemperatur des Gemisches. Beim Einsatz von 1 mmol (Chlorsilan)/ $g(SiO_2)$ kann bei 70 °C innerhalb von 2 Stunden eine vollständige Reaktion erreicht werden.

Je nach Menge Siloxan und Halogensilan wird ein Belegungsgrad von bis 90 % der SiOH-Gruppen auf der Oberfläche erreicht.

Kolloidales Silica hat üblicherweise ca. 4,6 SiOH-Gruppen pro $nm^2$.

**Verfahrensschritt (3)**

Die Entfernung von Wasser aus dem Kieselsol kann beispielsweise durch eine Extraktion mit einer Phasentrennung, eine Destillation, eine azeotrope Destillation oder durch ein Membranverfahren erfolgen.

In einer bevorzugten Ausführungsform des erfindungsgemäßen Verfahrens erfolgt die Entfernung von Wasser durch eine azeotrope Destillation mit einem organischen Lösemittel.

Die azeotrope Destillation bietet den Vorteil, dass das Wasser aus dem Kieselsolsystem entfernt werden kann, ohne dass dabei auf die Eignung des organischen Lösemittels zur Phasentrennung zu achten wäre. Da eine azeotrope Destillation im Allgemeinen mit fast allen organischen Lösemitteln, mit welchen Wasser ein Azeotrop bildet, gelingt, erhöht sich durch die azeotrope Destillation die Auswahlmöglichkeiten für das organische Lösemittel, so dass insgesamt ein Verfahren mit größerer Flexibilität resultiert.

Das organische Lösemittel, welches zur azeotropen Entfernung von Wasser aus dem Kieselsol verwendet wird, unterliegt keiner besonderen Beschränkung und es kann jedes beliebige Lösemittel verwendet wird, welches mit Wasser ein Azeotrop bildet. Bevorzugt ist dabei die Verwendung eines Lösemittels, welches zu einem wassermischbaren System aus Kieselsol und Lösemittel führt. Bevorzugt sind daher Lösemittel, welche sich mit Wasser im Wesentlichen vollständig, auch unter Verwendung von oberflächenaktiven Mitteln, mischen lassen.

Geeignete Lösemittel für die azeotrope Destillation können beispielsweise ausgewählt werden aus der Gruppe, bestehend aus Alkoholen, wie Methanol, Ethanol, n-Propanol, Isopropanol, Pentanole, Octanole und Cyclohexanol; Glycole, wie Ethylenglykol und Diethylenglykol; Ether, Glycol- und Propylenglykolether, wie Diethylether, Dibutylether, Anisol, 1,4-Dioxan, 1,3-Dioxan, 1,3-Dioxolan, Tetrahydrofuran, 1-Methoxy-2-propanol, 1-Methoxy-1-propanol, 2-Methoxyethanol, 1-Ethoxy-2-propanol, Mono-, Di-, Tri- und Polyethylenglycolether; Ketone und Aldehyde, wie Aceton, Butanon und Cyclohexanon; Ester, wie Essigsäureester und Glycolester; Amide und andere stickstoffhaltige Lösemittel, wie Dimethylformamid und Nitrobenzol, Piperidin, N-Methylpiperidin und Acetonitril; schwefelhaltige Lösemittel, wie Dimethylsulfoxid; Halogenkohlenwasserstoffe, wie Dichlormethan, Chloroform, Tetrachlormethan, Tri- und Tetrachlorethan, 1,2-Dichlorethan, Chlorfluorkohlenwasserstoffe; Kohlenwasserstoffe, wie Pentan, Hexan, Heptan, Benzine, Petrolether, Cyclohexan, Methylcyclohexan, Decalin, Terpene, Benzol, Toluol und Xylole; und dergleichen. Insbesondere bevorzugt ist Isopropanol.

Das erfindungsgemäße Verfahren kann darüber hinaus weitere optionale Verfahrensschritte, beispielsweise das Entfernen von flüchtigen Bestandteilen, wie von überschüssigen Silanen, umfassen, was vorzugsweise durch eine Destillation erfolgt.

In einer weiteren bevorzugten Ausführungsform der vorliegenden Erfindung wird die Modifizierung der Kieselsole in einem sauren pH-Wert durchgeführt, wobei es jedoch nicht möglich ist, den genauen sauren pH-Wert näher zu konkretisieren, da die erfindungsgemäße Umsetzung in einem organischen Lösemittel durchgeführt wird.

Die vorliegende Erfindung betrifft darüber hinaus die nach dem oben beschriebenen Verfahren erhältlichen Kieselsole.

Ein weiterer Gegenstand der vorliegenden Erfindung ist die Verwendung der erfindungsgemäßen Kieselsole bzw. der nach dem oben beschriebenen Verfahren erhältlichen Kieselsole. Die erfindungsgemäße Dispersion oder das aus der Dispersion durch Entfernen des Lösemittels gewonnene redispergierbare Pulver können in verschiedenste Basispolymere eingearbeitet werden und deren physikalische und insbesondere mechanische Eigenschaften verbessern bzw. verändern. Als Basispolymere können im Rahmen der Erfindung eine Vielzahl bekannter Polymere verwendet werden. Beispielsweise können mittels den erfindungsgemäßen Systemen thermoplastische oder duroplastische Kunststoffe modifiziert werden. Beispielhaft erwähnt seien Polyolefine, Polycarbonate, Polyamide, Polyimide, Polyacrylate, Polymethacrylate, Polyetherketone, Polysulfone, Polyurethane, Polyharnstoffe, Epoxidharze und Polyesterharze.. Modifizierbare Elastomere sind beispielsweise Naturkautschuk, Butylkautschuke, Acrylatkautschuke, Styrol-Butadien-Kautschuk (SBR), ggf. hydrierte Nitril-Butadien-Kautschuke, Polysiloxane (Silikone) etc. Bei vielen dieser Stoffgruppen ist es von besonderem Vorteil, die erfindungsgemäßen Nanopartikel als redispergierbares Pulver einzuarbeiten, da ein Einbringen über Lösungsmittel nachteilig und mit hohem Aufwand verbunden ist.

Besonders vorteilhaft kann das erfindungsgemäße nanoskalige Siliciumdioxid auch in Polymere bzw. Harze mit niedrigem Siedepunkt eingearbeitet werden, wie beispielsweise Methylmethacrylat (MMA).

Erfindungsgemäß hergestellte Partikel können ebenfalls zur Modifikation von Weichmachern wie beispielsweise Adipaten und Phthalaten verwendet werden. Sie bilden mit diesen Weichmachern niedrigviskose und stabile Dispersionen.

Die erfindungsgemäß hergestellten Partikeln enthaltende polymere bzw. polymerisierbare Mischungen stellen stabile und daher lagerfähige Dispersionen dar und weisen gute Fließeigenschaften (niedrige Viskosität, geringe Strukturviskosität) auf. Sie eignen sich daher beispielsweise für die Herstellung von Dentalformulierungen, die beispielsweise aus einem statischen Mischer appliziert werden und daher keine zu hohe Verarbeitungsviskositäten aufweisen dürfen. Besonders bevorzugt können sie bei Dentalformulierungen auf Basis von Silikonen verwendet werden. Ein anderes mögliches Anwendungsgebiet ist die Modifikation von LSRs (Liquid Silicone Rubber), die in der Regel im Spritzguss verarbeitet werden und bei denen deswegen eine niedrige Verarbeitungsviskosität von großem Vorteil ist. Erfindungsgemäß kann bei LSRs ein hoher Füllstoffgehalt und damit gute mechanische Eigenschaften des ausgehärteten Endprodukts erreicht werden, ohne dass die Verarbeitbarkeit durch eine zu hohe Viskosität darunter leidet.

Grundsätzlich ermöglicht es die Erfindung, polymerisierbare Mischungen bereitzustellen, die aufgrund ihrer niedrigen Viskosität gut verarbeitbar sind und als ausgehärtetes Polymer durch einen hohen Füllstoffgehalt bewirkte verbesserte Eigenschaften aufweisen, insbesondere mechanische Eigenschaften, verbesserte thermische Leitfähigkeit und dergleichen.

Aus den so erhaltenen Kieselsolen sowie den zuvor beschriebenen Kieselsolen lassen sich nach Entfernen des Lösemittels Pulver gewinnen, die in verschiedenen Medien redispergierbar sind. Dabei hat sich überraschenderweise herausgestellt, dass die Partikelgrößenverteilung nach der Dispergierung im wesentlichen der Partikelgrößenverteilung im Lösemittel entspricht, obwohl die Partikel bei der Trocknung agglomerieren, da das stabilisierende Medium wegfällt. Die Agglomeration ist jedoch im vorliegenden erfindungsgemäßen Fall im wesentlichen reversibel, so dass die Partikel mit einem geringen Energieaufwand wieder in eine Dispersion überführt werden können. Die Trocknung des oberflächenmodifizierten Kieselsols kann zum Beispiel durch die Sprühtrocknung erfolgen.

Der erfindungsgemäße Gegenstand weist eine Reihe an Vorteilen auf. So können durch die Variation des Alkoxysilans bei der ersten Modifizierung die Eigenschaften der Partikel unabhängig von den Halogensilanen und/oder den Siloxanen der zweiten Modifizierung eingestellt werden. Durch die Menge an Halogensilan bzw. Siloxan bei der zweiten Modifizierung kann wiederum die Polarität der resultierenden Kieselsolteilchen beeinflusst werden, da Kieselsolteilchen, welche nur mit einem Alkoxysilan beschichtet werden, im Allgemeinen relativ polar sind, während durch die zweite Modifizierung die Beschichtung insgesamt wieder unpolarer wird. Durch eine geschickte Kombination der Art und Menge der ersten und zweiten Modifizierung lassen sich Partikel herstellen, die in vorgegebenen Lösemitteln maßgeschneidert eine stabile Dispersion ergeben. Das erfindungsgemäße Verfahren erlaubt gewissermaßen eine Baukastenchemie zum gezielten Einstellen von Polarität und gleichzeitig eine Abschirmung der Oberfläche.

Da es sich bei der zweiten Modifizierung um eine Gleichgewichtsreaktion handelt, kann der Anteil an unpolaren Silylgruppen auf der Oberfläche über die Menge an Silan bei der Reaktion gezielt eingestellt werden.

Die erfindungsgemäßen bzw. nach dem erfindungsgemäßen Verfahren erhältlichen Kieselsolen können zur Herstellung redispergierbarer Pulver verwendet werden.

Durch die Verwendung der erfindungsgemäßen Kieselsole lassen sich die mechanischen Eigenschaften, insbesondere die Zugfestigkeit, das E-Modul, die Weiterreißfestigkeit, das Biegemodul und die Schlagzähigkeit, in Elastomeren, Verbundwerkstoffen und thermoplastischen Werkstoffen verbessern. Bei der Verwendung der erfindungsgemäßen Kieselsole in der Herstellung z.B. von optischen Linsen lassen sich höhere Brechungsindizes erreichen. Auch die Gasbarriereeigenschaften, das Brandverhalten sowie die Fließeigenschaften werden durch die erfindungsgemäßen Kieselsoldispersionen verbessert.

Darüber hinaus können die erhaltenen oberflächenmodifizierten Kieselsole in Dispersionsform beispielsweise zur Herstellung von Verbundstoffen (Nanocomposite) verwendet werden. Demnach sind ein weiterer Gegenstand der Erfindung die mit den erfindungsgemäßen Kieselsolen erhältlichen Verbundstoffe (Nanocomposites). Diese sind aufgrund ihrer verbesserten mechanischen Eigenschaften, z.B. erhöhte Kratz- und Abriebfestigkeit (Tribologie), vorteilhaft. Dies gilt beispielsweise für die Verwendung in Lacken.

Die vorliegende Erfindung wird durch die folgenden Beispiele näher beschrieben, welche die vorliegende Erfindung jedoch nicht beschränken.

### Beispiele:

### Methode zur Teilchengrößenbestimmung

Die Teilchengröße kann in Lösung mittels dynamischer Licht-streuung (DLS) auf einem "Dynamic Light Scattering Particle Size Analyzer LB-550" der Firma Horiba bei einer Konzentration von maximal 10 Gew.-% Partikeln erfolgen, wobei die Dispersion dazu maximal eine dynamische Viskosität von 3 mPas bei 25 °C aufweisen sollte. Als Teilchengröße wird der Median (D50-Wert) der Partikelgrößenverteilung angeben.

Im Feststoff kann die Teilchengröße durch Transmissionselektronenmikroskopie bestimmt werden. Dazu werden mindestens 100 Partikel ausgemessen und eine Partikelgrößenverteilung gebildet.

### Bestimmung der Oberfläche

Die Oberfläche wird auf Basis der Partikelgröße berechnet. Dabei wird davon ausgegangen, dass die Partikel alle den gleichen Durchmesser entsprechend des Medians ($d_{50}$-Wert) der Partikelgrößenverteilung und eine sphärische Form aufweisen.

Für die spezifische Oberfläche ($nm^2$/g (Partikel)) gilt:

$$A_0 = 6 / (\rho \times d_{50}),$$

wobei $\rho$ die Dichte der Partikel ist (Dichte ($SiO_2$) = 2,1 g/cm$^3$).

Die Zahl der Gruppen N ergibt sich aus:

$$N = ([Mol \text{ (reaktive Gruppen)}] / [\text{Masse der Partikel}]) \times 6,022 \times 10^{23}$$

Der Quotient ($N / A_0$) ergibt die Zahl der Gruppen pro Oberflächeneinheit.

Bei der Reaktion von Alkoxysilanen kann vereinfachend angenommen werden, dass das eingesetzte Silan vollständig auf der Oberfläche der Partikel hydrolisiert.

### Vergleichsbeispiele

Ein basisches kolloidales Kieselsol (40 Gew.-% $SiO_2$ in Wasser; mittlere Teilchengröße $d_{50}$ (bestimmt mittels dynamischer Lichtstreuung): 25 nm; stabilisiert mit NaOH) wurde über einen sauren Ionenaustauscher (Amberjet 1200H, erhältlich von Rohm & Haas) gerührt, bis ein pH-Wert von 2 bis 3 erreicht war. Nachdem der Ionenaustauscher durch Filtration entfernt war, wurde das saure Sol mit verschiedenen Alkoxysilanen (siehe Ziffern 2 bis 5 der Tabellen 1 und 2 unten) 90 Minuten lang gerührt. Das Beispiel 1 wurde ohne Alkoxysilan durchgeführt und diente daher zum Vergleich.

Das Sol wurde anschließend mit Isopropanol verdünnt und unter Zugabe von weiterem Isopropanol wurde das Gemisch von Lösemittel und Wasser bei reduziertem Druck abdestilliert. Das erhaltene Sol wurde unter Rühren mit Chlortrimethylsilan und Hexamethyldisiloxan versetzt. Das Gemisch wurde zwei Stunden bei 70 °C gerührt, durch Zugabe von Amberjet 4400 OH neutralisiert und der Ionenaustauscher abfiltriert

### Ergebnisse

Zum Vergleich der Eigenschaften der Partikel mit unterschiedlichen Alkoxysilanen (siehe Ziffern 2 bis 5 in den Tabellen 1 und 2) in der ersten Beschichtung wurden Kieselsole bei 40 °C im Vakuum getrocknet. Die resultierenden Pulver wurden in Toluol redispergiert, so dass Sole mit 10 Gew.-% Feststoffanteil entstanden. Diese wurden über dynamische Lichtstreuung vermessen.

**Tabelle 1**

| Beispiel | Teilchengröße $d_{50}$ [nm] | Spanne $(d_{90}-d_{10})/(d_{50})$ | Viskosität [mPas] |
|---|---|---|---|
| 1) Kein Alkoxysilan | 696 | 4,0 | 20 |
| 2) Propyltrimethoxysilan | 30,3 | 0,7 | 0,9 |
| 3) Octyltrimethoxysilan | 28,0 | 0,7 | 0,7 |

Die Auswertung der Tabelle 1 zeigt, dass je näher die gemessene Partikelgröße an der ursprünglich vorhandenen Partikelgröße und -verteilung liegt, desto besser sind die Partikel geeignet, in Toluol redispergiert zu werden. An den Ergebnissen in Tabelle 1 lässt sich erkennen, dass das im ersten Verfahrensschritt eingesetzte Alkoxysilan die Redispergierbarkeit der Partikel in Toluol erheblich verbessert. Auch die Viskosität der Dispersionen ist ein Maß für die Verträglichkeit der Partikel zu der Matrix (Lösemittel). Die mit Alkoxysilan umgesetzten Partikel verursachen in Toluol eine erheblich niedrigere Viskosität als die Partikel ohne Alkoxysilan, d.h. sie sind mit Toluol besser verträglich.

Werden unterschiedliche Kieselsole über Lösemittelaustausch in Toluol überführt und mittels DLS vermessen, kann man die Polarität der Partikel anhand eines Vergleichs mit dem ursprünglichen Isopropanolsol ablesen.

**Tabelle 2**

| Alkoxysilan | Teilchengröße $d_{50}$ in Isopropanol [nm] | Teilchengröße $d_{50}$ in Toluol [nm] |
|---|---|---|
| 4) Phenyltrimethoxysilan | 104 | 46 |
| 5) $\gamma$-Methacryloxypropyltrimethoxysilan | 44 | 4470 |

Die Verträglichkeit von Partikeln mit Lösemitteln lässt sich an der Partikelgröße in der dynamischen Lichtstreuung ablesen. Partikel, die mit dem Lösemittel nicht verträglich sind, lagern sich zusammen und erscheinen in der dynamischen Lichtstreuung bei größerer Teilchengröße. Je näher die gemessenen Teilchengrößen an der tatsächlichen Teilchengröße liegt, desto weniger Partikel sind also zusammengelagert.

Die Ergebnisse aus Tabelle 2 machen deutlich, dass die Partikel, die mit dem γ-Methacryloxypropyltrimethoxysilan umgesetzt wurden, mit dem polareren Lösemittel Isopropanol besser verträglich sind, als mit dem unpolareren Toluol. Die polare γ-Methacryloxypropyl-Gruppe ermöglicht eine gute Verträglichkeit mit unpolaren Lösemitteln. Diese Ergebnisse zeigen, dass die vorliegende Erfindung im Sinne einer "Baukastenchemie" zum gezielten Anbringen gewünschter Moleküle mittels Silylierung dienen kann.

**Vorschrift für die Beispiele 1 - 3 (Tabelle 1)**

Ein basisches kolloidales Kieselsol (40 Gew.-% $SiO_2$ in Wasser, Teilchengröße (DLS) D50 = 25 nm, stabilisiert mit NaOH) wurde mit saurem Ionentauscher Amberjet 1200 H (Rohm & Haas) gerührt, bis ein pH-Wert von 2 erreicht war. 100 Teile des Sols wurden mit 0,24 mmol des Alkoxysilans / Teil(Sol) für 2 h gerührt (außer bei Beispiel 1). Dann wurde mit 600 Teilen Isopropanol verdünnt und das Sol bei 40 - 50 °C im Vakuum auf ca. 150 Teile eingeengt.

Das Sol wurde durch Zugabe von Isopropanol auf 300 Gewichtsteile aufgefüllt und dann mit einem Gemisch aus 4,4 Teilen Chlortrimethylsilan und 13 Teilen Hexamethyldisiloxan versetzt und 2 Stunden bei 70 °C gerührt. Nach dem Entfernen der Heizung wurden 25 Teile Amberjet 4400 OH (basischer Ionentauscher, Rohm & Haas) dazu geben. Nach einer weiteren Stunde Rühren wurde der Ionentauscher abfiltriert.

**Vorschrift für die Beispiele 4 und 5 (Tabelle 2)**

Ein basisches kolloidales Kieselsol (40 Gew.-% $SiO_2$ in Wasser, Teilchengröße (DLS) D50 = 25 nm, Stabilisiert mit NaOH) wurde mit saurem Ionentauscher Amberjet 1200 H (Rohm & Haas) gerührt, bis ein pH-Wert von 2 erreicht war. 100 Teile des Sols wurden mit 0,24 mmol des Alkoxysilans / Teil(Sol) für 2 h gerührt. Dann wurde mit 600 Teilen Isopropanol verdünnt und das Sol bei 40 - 50 °C im Vakuum auf ca. 150 Teile eingeengt.

Das Sol wurde durch Zugabe von Isopropanol auf 160 Gewichtsteile aufgefüllt. Dann wurde mit einem Gemisch aus 4,4 Teilen Chlortrimethylsilan und 13 Teilen Hexamethyldisiloxan versetzt und 2 Stunden bei 70 °C gerührt. Nach dem Entfernen der Heizung wurden 25 Teile Amberjet 4400 OH (basischer Ionentauscher, Rohm & Haas) dazu geben. Nach einer weiteren Stunde Rühren wurde der Ionentauscher abfiltriert.

**Patentansprüche**

1. Oberflächenmodifiziertes Siliziumdioxid-Partikel, **dadurch gekennzeichnet, dass** es eine Oberfläche mit der folgenden Belegung aufweist:

   (a) 0,1 bis 16 Gruppen/$nm^2$ der Art (Modifizierung vom Typ A)

   $$(Oberfläche\text{-}SiO)_x\text{-}Si(R^1)_y(OR^2)_{4-x-y}$$

   mit x = 1 bis 3, y = 1 bis 3 und x + y = 2 oder 3; und
   (b) 0,1 bis 16 Gruppen/$nm^2$ der Art (Modifizierung vom Typ B)

   $$(Oberfläche\text{-}SiO)_z SiR^3_{4-z}$$

   mit z = 1 oder 2

   aufweist, wobei die Reste $R^1$, $R^2$ und $R^3$ beliebige organische Reste darstellen können und mehrere Reste $R^1$, $R^2$ oder $R^3$ gleich oder verschieden sein können.

2. Soliziumdioxid-Partikel nach Anspruch 1, **dadurch gekennzeichnet das** A ungleich B ist.

3. Siliziumdioxid-Partikel nach Anspruch 1 oder 2, **dadurch gekennzeichnet, dass** es eine Modifizierung der Art $(Oberfläche\text{-}SiO)_x\text{-}Si(R)_y(OR)_{4-x-y}$ (Modifizierungsart vom Typ A) von 0,1 bis 10, insbesondere von 0,15 bis 6, besonders bevorzugt 0,2 bis 4 Gruppen/$nm^2$ aufweist.

**4.** Siliziumdioxid-Partikel nach einem der Ansprüche 1 bis 3, **dadurch gekennzeichnet, dass** es eine Modifizierung der Art (Oberfläche-SiO)$_z$-SiR$^3_{4-z}$ (Modifizierungsart vom Typ B) von 0,2 bis 10, insbesondere von 0,3 bis 6, besonders bevorzugt 0,4 bis 4 Gruppen/nm$^2$ aufweist.

**5.** Kieselsol, **dadurch gekennzeichnet, dass** es ein Siliziumdioxid-Partikel nach einem der Ansprüche 1 bis 4 aufweist.

**6.** Verfahren zur Herstellung von oberflächenmodifizierten Siliziumdioxid-Partikeln oder Kieslsol durch Umsetzung von Kieselsol mit mindestens einem ersten Modifizierungsmittel, umfassend mindestens ein Alkoxysilan, und mindestens einem zweiten Modifizierungsmittel, ausgewählt aus der Gruppe bestehend aus Halogensilan, Siloxan und Mischungen davon.

**7.** Verfahren nach Anspruch 6, **dadurch gekennzeichnet, dass** das Verfahren die Verfahrensschritte

(1) der Umsetzung von kolloidalem Kieselsol mit mindestens einem ersten Modifizierungsmittel, umfassend mindestens ein Alkoxysilan,
(2) der Umsetzung von kolloidalem Kieselsol mit mindestens einem zweiten Modifizierungsmittel, ausgewählt aus einem Halogensilan, einem Siloxan und Mischungen davon, sowie
(3) die Entfernung von Wasser aus dem Kieselsol, insbesondere durch azeotrope Destillation,

umfasst.

**8.** Verfahren nach Anspruch 6 oder 7, **dadurch gekennzeichnet, dass** die Umsetzung mit dem ersten und dem zweiten Modifizierungsmittel entweder aufeinanderfolgend oder gleichzeitig mit einer Mischung aus dem ersten und dem zweiten Modifizierungsmittel erfolgt.

**9.** Verfahren nach einem der Ansprüche 7 bis9, **dadurch gekennzeichnet, dass** bei der zweistufigen Modifizierung der Oberfläche das Wasser vor der ersten Oberflächenmodifizierung oder zwischen der ersten und der zweiten Oberflächenmodifizierung aus dem Reaktionssystem entfernt wird.

**10.** Verfahren nach einem der Ansprüche 6 bis 9, **dadurch gekennzeichnet, dass** das Alkoxysilan der allgemeinen Formel (I)

$$\text{Formel (I)} \qquad R^1_x Si(OR^2)_{4-x},$$

entspricht, in welcher der Rest $R^1$ einem gegebenenfalls substituierten $C_1$-$C_{18}$-Alkylrest entspricht und der Rest $R^2$ ausgewählt ist aus der Gruppe, bestehend aus einem gegebenenfalls substituierten $C_1$-$C_{18}$-Alkylrest, einem Carboxyrest, einem gegebenenfalls substituierten $C_2$-$C_{18}$-Alkenylrest und einem Oximrest und der Rest $R^1$.

**11.** Verfahren nach einem der Ansprüche 6 bis 10, **dadurch gekennzeichnet, dass** das Halogensilan der allgemeinen Formel (II)

$$\text{Formel (II)} \qquad R^3_a H_b SiX_{4-a-b}$$

entspricht, in welcher
jedes $R^3$, unabhängig voneinander, ausgewählt ist aus der Gruppe, bestehend aus Kohlenwasserstoffresten mit 1 bis 18 Kohlenstoffatomen oder organofunktionellen Kohlenwasserstoffresten mit 1 bis 18 Kohlenstoffatomen;
X, unabhängig voneinander, ausgewählt ist aus der Gruppe, bestehend aus Fluor, Chlor, Brom und Iod;
a gleich 0, 1, 2 oder 3 ist;
b gleich 0 oder 1 ist; und
a + b = 1, 2 oder 3 ist.

**12.** Verfahren nach einem der Ansprüche 6 bis 11, **dadurch gekennzeichnet, dass** das Siloxan der allgemeinen Formel (III)

$$\text{Formel (III)} \qquad R^3_n SiO_{(4-n)/2}$$

entspricht, in welcher
jedes $R^3$, unabhängig voneinander, ausgewählt ist aus der Gruppe, bestehend aus Kohlenwasserstoffresten mit 1 bis 18 Kohlenstoffatomen, organofunktionellen Kohlenwasserstoffresten mit 1 bis 18 Kohlenstoffatomen, einem Wasserstoffatom und einer OH-Gruppe; und

n eine Zahl zwischen 2 und einschließlich 3 ist.

13. Verfahren nach einem der Ansprüche 6 bis 12, **dadurch gekennzeichnet, dass** das erhaltene Kieselsol getrocknet wird.

14. Kieselsol, erhältlich durch ein Verfahren gemäß einem der Ansprüche 6 bis 13.

15. Oberflächenmodifiziertes Siliziumdioxid, erhältlich durch ein Verfahren gemäß einem der Ansprüche 6 oder 13.

16. Verwendung eines Siliziumdioxids-Partikels oder Kieselsols nach einem der Ansprüche 1 bis 5, 14 oder 15 in Basispolymeren, beispielsweise Polyolefinen, Polycarbonaten, Polyamiden, Polyimiden, Polyacrylaten, Polymethacrylaten, Polyetherketonen, Polysulfonen, Polyurethanen, Polyharnstoffen, Epoxidharzen, Polyesterharzen, Polysiloxanen Naturkautschuk, Butylkautschuken, Acrylatkautschuken, Styrol-Butadien-Kautschuken (SBR), ggf. hydrierten Nitril-Butadien-Kautschuken und Methylmethacrylat (MMA).

**Europäisches Patentamt**

**EUROPÄISCHER RECHERCHENBERICHT**

Nummer der Anmeldung

EP 08 00 7625

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2004/052939 A1 (BOSWELL LISA MARIE [US] ET AL) 18. März 2004 (2004-03-18) * Beispiele 3-5 * * Absätze [0006] - [0011] * | 1-16 | INV. C09C1/30 C08K9/06 |
| X,D | EP 0 982 268 A (DOW CORNING [US]) 1. März 2000 (2000-03-01) * Absätze [0008] - [0028] * * Beispiel 1 * | 1-16 | |
| X | US 3 015 645 A (TYLER LESLIE J) 2. Januar 1962 (1962-01-02) * Beispiele 2,3 * * Tabelle 1 * * Spalte 1, Zeile 54 - Spalte 4, Zeile 10 * | 1-16 | |
| X | US 6 706 398 B1 (REVIS ANTHONY [US]) 16. März 2004 (2004-03-16) * Spalte 1, Zeile 37 - Spalte 3, Zeile 25 * * Beispiele 1-10 * | 1-16 | |
| | | | RECHERCHIERTE SACHGEBIETE (IPC) |
| X | US 5 942 590 A (BURNS GARY THOMAS [BE] ET AL) 24. August 1999 (1999-08-24) * Beispiel 4 * * Spalte 4, Zeile 23 - Spalte 8, Zeile 50 * | 1-16 | C09C C08K |
| X | US 6 107 351 A (BURNS GARY THOMAS [BE] ET AL) 22. August 2000 (2000-08-22) * Spalte 3, Zeile 24 - Spalte 8, Zeile 60 * * Beispiel 2 * | 1-16 | |
| X A | EP 1 142 917 A (CLARIANT FRANCE SA [FR]) 10. Oktober 2001 (2001-10-10) * Absätze [0008] - [0036] * * Beispiel 1 * | 1,3-5, 14-16 2,6-13 | |

Der vorliegende Recherchenbericht wurde für alle Patentansprüche erstellt

| Recherchenort | Abschlußdatum der Recherche | Prüfer |
|---|---|---|
| Den Haag | 4. September 2008 | Marino, Emanuela |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**                    EP 08 00 7625

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-09-2008

| Im Recherchenbericht angeführtes Patentdokument | | Datum der Veröffentlichung | Mitglied(er) der Patentfamilie | | Datum der Veröffentlichung |
|---|---|---|---|---|---|
| US 2004052939 | A1 | 18-03-2004 | KEINE | | |
| EP 0982268 | A | 01-03-2000 | DE | 69906895 D1 | 22-05-2003 |
| | | | DE | 69906895 T2 | 12-02-2004 |
| | | | JP | 2000080201 A | 21-03-2000 |
| | | | US | 6051672 A | 18-04-2000 |
| US 3015645 | A | 02-01-1962 | KEINE | | |
| US 6706398 | B1 | 16-03-2004 | AT | 381591 T | 15-01-2008 |
| | | | AU | 2003265979 A1 | 30-04-2004 |
| | | | CN | 1681880 A | 12-10-2005 |
| | | | EP | 1546250 A2 | 29-06-2005 |
| | | | JP | 2005539112 T | 22-12-2005 |
| | | | KR | 20050054938 A | 10-06-2005 |
| | | | WO | 2004024812 A2 | 25-03-2004 |
| US 5942590 | A | 24-08-1999 | AU | 6330198 A | 09-09-1998 |
| | | | BR | 9807855 A | 22-02-2000 |
| | | | CA | 2280901 A1 | 27-08-1998 |
| | | | CN | 1248221 A | 22-03-2000 |
| | | | DE | 69801742 D1 | 25-10-2001 |
| | | | DE | 69801742 T2 | 04-07-2002 |
| | | | EP | 0963343 A1 | 15-12-1999 |
| | | | ES | 2165147 T3 | 01-03-2002 |
| | | | JP | 3365639 B2 | 14-01-2003 |
| | | | JP | 2000512973 T | 03-10-2000 |
| | | | WO | 9837013 A1 | 27-08-1998 |
| US 6107351 | A | 22-08-2000 | AU | 6178498 A | 09-09-1998 |
| | | | BR | 9807718 A | 15-02-2000 |
| | | | CA | 2280909 A1 | 27-08-1998 |
| | | | CN | 1248220 A | 22-03-2000 |
| | | | DE | 69813191 D1 | 15-05-2003 |
| | | | DE | 69813191 T2 | 05-02-2004 |
| | | | EP | 0963342 A1 | 15-12-1999 |
| | | | JP | 3394048 B2 | 07-04-2003 |
| | | | JP | 2000512974 T | 03-10-2000 |
| | | | TW | 467867 B | 11-12-2001 |
| | | | WO | 9837014 A1 | 27-08-1998 |
| EP 1142917 | A | 10-10-2001 | CA | 2337569 A1 | 03-10-2001 |
| | | | DE | 1142917 T1 | 28-05-2003 |
| | | | FR | 2807052 A1 | 05-10-2001 |
| | | | JP | 2001302734 A | 31-10-2001 |
| | | | US | 2001027223 A1 | 04-10-2001 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0982268 A
- US 6736891 B
- US 2801185 A
- US 2786042 A
- EP 0982268 B1

**In der Beschreibung aufgeführte Nicht-Patentliteratur**

- **Ralph K. Iler.** The Chemistry of Silica. John Wiley & Sons, Inc, 1979
- **J. Huheey.** Anorganische Chemie. Walter de Gruyter, 1988, 309f